# EUROPEAN PATENT APPLICATION

(11) **EP 1 481 642 A1**
(43) Date of publication of application: **01.12.2004**
(21) Application number: 04012579.1
(22) Date of filing: 27.05.2004
(51) Int. Cl.: A61B 18/14

(54) **Forceps for endoscope**

(30) Priority: 29.05.2003 JP 2003152605
(71) Applicant: Olympus Corporation, Shibuya-ku, Tokyo (JP)
(72) Inventor: Suzuki, Keita, Tokyo (JP)
(74) Representative: von Hellfeld, Axel, Dr. Dipl.-Phys.

(57) **Abstract**

A high-frequency current forceps (10) has a pair of clamp pieces (12, 13) facing each other. Each clamp piece has an electrically insulated clamp face facing each other. The clamp piece (13) has a body (13a) on which a surface is electrically insulated, and a wire (18) having two ends supported on the body. A concave portion (13c) is formed on the center portion of the clamp face so that the center portion forms a curved surface protruding toward a backside of the clamp piece in relation to its clamp face. Each end of the wire is installed inside the inner wall of the concave portion. The wire is supported above the concave portion so that the wire contacts another clamp piece (12) when the two clamp pieces are closed.

## Description

### BACKGROUND OF THE INVENTION

Priority is claimed on Japanese Unexamined Patent Application, First Publication No. 2003-152605, filed May 29, 2003, the content of which is incorporated herein by reference.

### Field of the Invention

The present invention relates to a forceps for an endoscope which performs treatments such as making an incision in a tissue by inserting it into a living organ and supplying high-frequency current on the tissue.

### Description of Related Art

A forceps for an endoscope is used for various kinds of treatments to excise tissue together with an endoscope. A high-frequency current forceps for excising a tissue by clamping the tissue using a tip portion of the forceps and by supplying high-frequency current to the tissue, is known as one of such forceps for an endoscope.

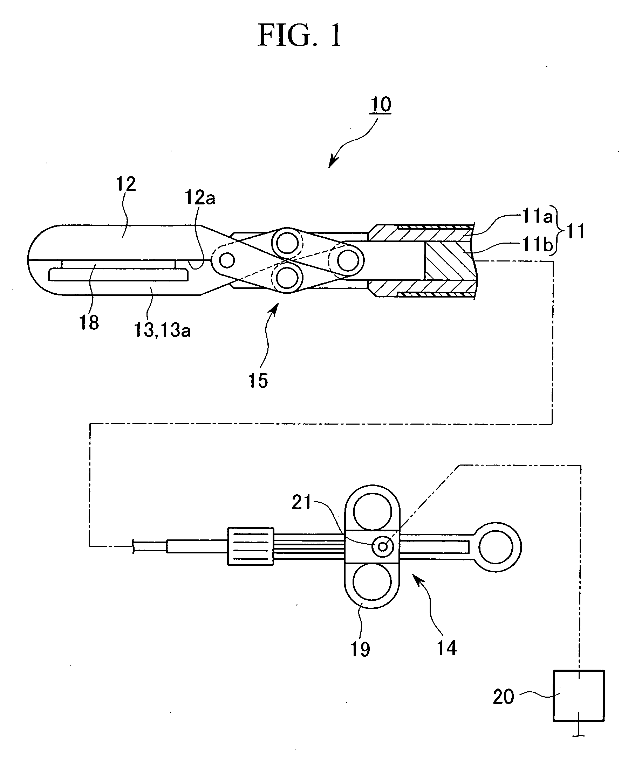

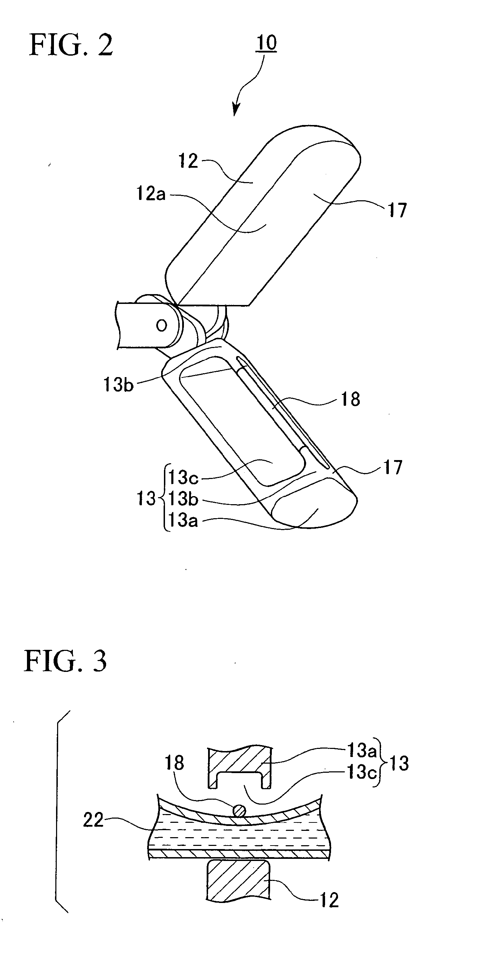

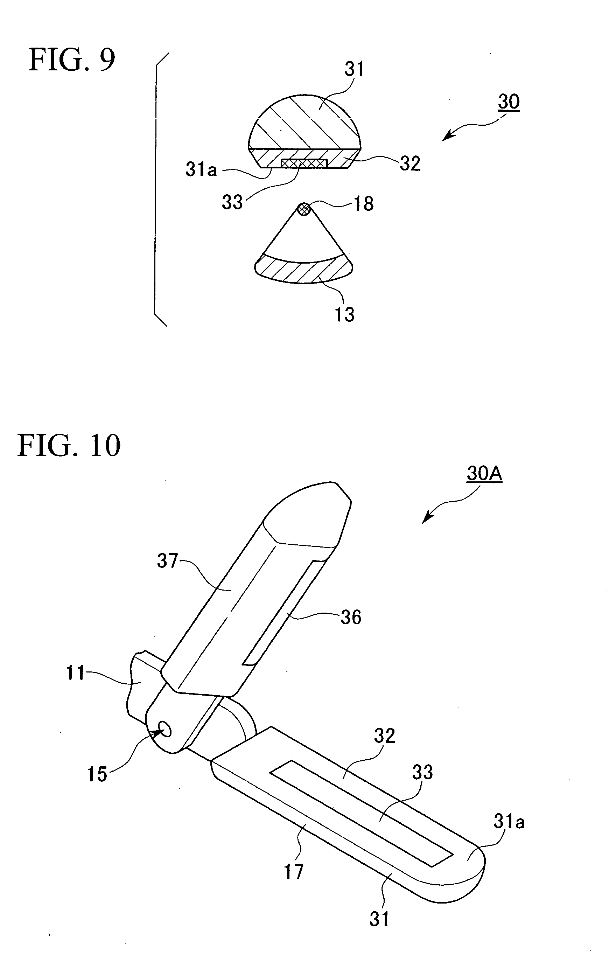

Conventionally, a forceps having electrodes on each of an insulated clamp piece and a clamp face facing to the clamp piece (for example, refer to FIG. 2 of Japanese Unexamined Patent Application, First Publication No. Hei 5-253241), a forceps having insulated scissors-type clamp pieces and electrodes on each clamp face of the clamp pieces facing to each other (for example, refer to FIGS. 9 and 10 of U. S. Patent 5,827,281), and a forceps having needle-shaped electrodes (for example, refer to FIG. 1 of Japanese Unexamined Patent Application, First Publication No. Hei 8-299355), are proposed as the high-frequency current forceps.

Furthermore, as for a forceps which is not used with an endoscope, a forceps which cauterizes or excises a living organ by exposing its wire-shaped electrodes, is also proposed (for example, refer to FIG. 5 of the specification of the PCT International Publication No. WO01/28444).

### SUMMARY OF THE INVENTION

A forceps for an endoscope of the present invention includes a pair of clamp pieces, wherein at least one of the clamp pieces includes: an electrically insulated body on which a concave portion is formed on a clamp face of the body; and a linear electrode having two ends supported above the concave portion.

The concave portion may be formed between a tip side portion and a bottom side portion of the body; and the two ends of the linear electrode may be supported on the tip side portion and the bottom side portion.

A ridgeline extending between the tip side portion and the bottom side portion except for the concave portion may be formed on the clamp face, and the two ends of the linear electrode may be supported on the ridgeline.

A wire may be used as the linear electrode.

Another forceps for an endoscope according to the present invention includes a pair of clamp pieces, wherein at least one of the clamp pieces comprises: an electrically insulated body on which a plurality of concave portions are formed on a clamp face of the body along a crossing direction in relation to a direction extending between a tip side portion and a bottom side portion of the clamp face; and a linear electrode which is supported above the concave portions.

A wire may be used as the linear electrode.

### BRIEF DESCRIPTION OF THE DRAWINGS

FIG. 1 shows a high-frequency current forceps which is a first embodiment of a forceps for an endoscope according to the present invention.
FIG. 2 shows clamp pieces of the high-frequency current forceps.
FIG. 3 shows a tissue clamped by the high-frequency current forceps.
FIG. 4 shows another example of clamp pieces of the high-frequency current forceps.
FIG. 5 shows clamp pieces according to a second embodiment of the present invention.
FIG. 6 shows another example of clamp pieces of the high-frequency current forceps according to the present invention.
FIG. 7 is a perspective view of the high-frequency current forceps according to the related technology of the present invention.
FIG. 8 is a plan view of the high-frequency current forceps according to the related technology of the present invention.
FIG. 9 is a cross sectional view of clamp pieces of the high-frequency current forceps shown in FIG. 8.
FIG. 10 is a perspective view of the high-frequency current forceps according to the related technology of the present invention.
FIG. 11 is a cross sectional view of clamp pieces of the high-frequency current forceps shown in FIG. 10.
FIG. 12 is a side view of a forceps according to related technology of the present invention.
FIG. 13 is an enlarged cross-sectional view of one part of the forceps shown in FIG. 12.

### DETAILED DESCRIPTION OF THE INVENTION

A first embodiment of a forceps for an endoscope according to the present invention will be explained below referring to FIGS. 1 to 3.

As shown in FIG. 1, a high-frequency current forceps (the forceps for an endoscope) 10 according to the present embodiment has a flexible shaft part 11 inserted in a canal of an endoscope (which is not shown in the figures). A pair of clamp pieces 12 and 13 which face each other and extend along an axis of the shaft part 11 and clamps a living organ to be clamped, is provided on a tip side of the shaft part 11. A control part 14 is provided on a bottom end side of the shaft member 11.

The shaft part 11 has a flexible tube 11a and a control wire 11b inserted in the flexible tube 11a. A tip end of the control wire 11b is connected to the pair of clamp pieces 12 and 13 via a link mechanism 15. An outer periphery of the flexible tube 11a is covered with an electrical insulation cover.

The clamp piece 12 is made of a metal such as a stainless steel, and the surface is covered with an insulation film 17 having an electric non-conductance. The clamp piece 12 has a clamp face 12a facing to the clamp piece 13.

As shown in FIG. 2, the clamp piece 13 has a body 13a which is made of metal such as a stainless steel and is covered with the insulation film 17 having an electric non-conductance, and a wire 18 held by the body 13a.

A ridgeline 13b is formed on the middle portion of the body 13a extending along the length direction of the clamp piece 13. In addition, a large concave portion 13c is formed on the body 13a by removing a middle portion except a tip side portion and a portion close to the shaft member 11.

Two ends of the wire 18 are supported by brazing them on the body 13a along the ridgeline 13b of the body 13a. Furthermore, the wire 18 is held over the concave portion 13c along the ridgeline 13b.

One end of the wire 18 is electrically connected to the flexible tube 11 a or the control wire 11b via a lead wire (which is not shown in the figures).

The control part 14 has a sliding controller 19 to which one end of the control wire 11b is connected, and a connection plug 21 for electrically connecting between the wire 18 and one of the electrodes of a high-frequency current power supply 20. Another electrode of the high-frequency current power supply 20 (which is not shown in the figures) is connected to a skin of a human body so that a connection area between them is sufficiently larger than a connection area between the skin and the wire 18.

Next, use of the high-frequency current forceps 10 according to the present embodiment having the above-mentioned constitution will be explained referring to FIG. 3.

Firstly, an endoscope not shown in the figures is inserted into a body cavity of a human body. Then, an injection needle (which is not shown in the figures) is inserted into the body cavity through the high-frequency current forceps 10, and a treatment part 22 to be excised is enlarged by injecting physiological saline solution into a lower layer of a mucous membrane of the treatment part 22. After that, the high-frequency current forceps 10 is inserted into the body cavity through the endoscope. At this time, the sliding controller 19 maintains its backward position, and the pair of clamp pieces 12 and 13 maintains their closed state.

Next, the high-frequency current forceps 10 is operated. By moving the sliding controller 19 toward the forward position, the link mechanism 15 is driven via the control wire 11b, and then the pair of clamp pieces 12 and 13 open. Then, after applying the clamp face 12a and the wire 18 on the enlarged treatment part 22, the sliding controller 19 is again pulled backward. Then, the link mechanism 15 is driven in opposite directions, and the pair of clamp pieces 12 and 13 close. The wire 18 makes a line-contact to the treatment part 22 and presses it, then clamps a living organ of the treatment part 22 together with the clamp face 12a. At this time, physiological saline solution, etc., around the living organ diverges through the concave portion 13c without remaining.

In this condition, when high-frequency current is supplied to the wire 18 by controlling the high-frequency current power supply 20, the high-frequency current is supplied to the another electrode (not shown in the figures) pasted to the human body, through the human body. At this time, current having very high electric current density flows near the wire 18 because a contact area between the wire 18 and the living organ is sufficiently small in relation to a size of the human body. As a result, the living organ contacting to the wire 18 is excised.

In addition, because there is a concave portion 13c around the wire 18, physiological saline solution, etc., will not remain around the wire 18, therefore lowering of the electric current density due to divergence of current can be prevented.

After the incision, the treatment part 22 is removed by removing the endoscope from the body cavity, maintaining the treatment part 22 clamped.

According to the high-frequency current forceps 10, because the wire 18 is used as an electrode, the surface area of the wire 18 can easily be made smaller by adjusting its external diameter, and therefore the electric current density can be increased. In addition, incision of the treatment part 22 can be done in a short time by concentrating the electric current density much more. Furthermore, because the two ends of the wire 18 are supported on the ridgeline 13b of the body 13a, only the treatment part 22 facing the clamp face 12a can contact the wire 18.

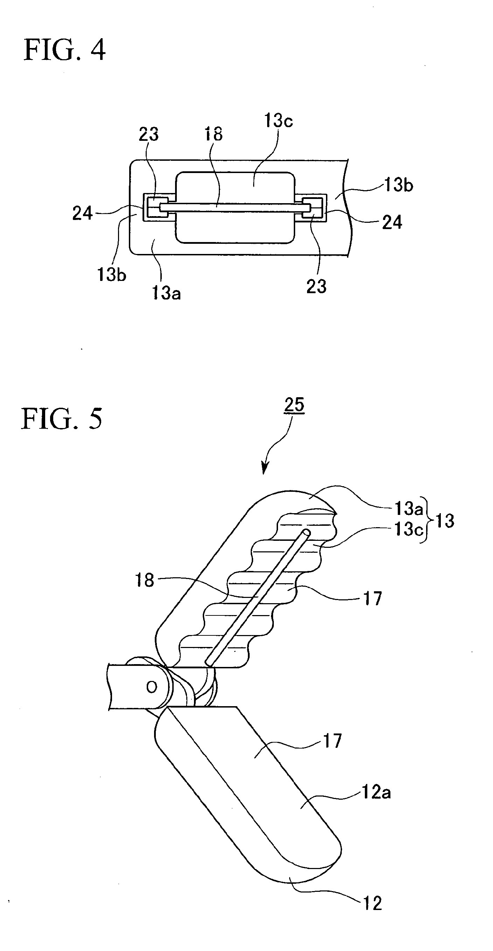

Moreover, in the present embodiment, the wire 18 is fixed by brazing the two ends thereof on the ridgeline 13b; however, the wire 18 may be fixed by an adhesive. Furthermore, as shown in FIG. 4, the wire 18 may be fixed by clamping the two ends with insulation members 23, and then inserting the two ends into supporting members 24 arranged on each end of the ridgeline 13b.

Next, a second embodiment of a forceps for an endoscope according to the present invention will be explained below referring to FIG. 5. In the explanation below, as for the same components explained in the first embodiment, the same reference numbers will be used and explanation thereof will be omitted.

A high-frequency current forceps (a forceps for an endoscope) 25 according to the present embodiment differs from the high-frequency current forceps 10 according to the above first embodiment in the point that a plurality of concave portions 13c are formed instead of one large concave portion 13c formed on a location on the body 13a where the ridgeline 13b exists.

The concave portions 13c are formed extending along a direction perpendicular to a length direction of the clamp piece 13 (a crossing direction in relation to a direction extending between a tip side portion and a bottom side portion of the clamp piece 13). Furthermore, the plurality of concave portions 13c are formed so that they are connected to each other in alignment along the length direction of the clamp piece 13.

The wire 18 is supported on the clamp piece 13, crossing above of the concave portions 13c by extending toward the length direction of the clamp piece 13.

Next, use of the high-frequency current forceps 25 will be explained.

As same as the high-frequency current forceps 10 according to the first embodiment, an endoscope (not shown in the figures) having this high-frequency current forceps 25 is inserted into a body cavity. Next, the clamp pieces 12 and 13 clamp the treatment part 22 by controlling the sliding controller 19. At this time, as in the first embodiment, physiological saline solution, etc., does not remain around the wire 18. Therefore, it becomes possible to conduct a treatment, reducing the likelihood of dispersion of current density due to electrical leakage via the physiological saline solution, etc., to a living organ except for the treatment part 22, and the likelihood of electrical damage on the electrical leakage portion. Furthermore, even a sticky living organ can be firmly clamped without slipping because the concave portions 13c can increase the surface area of the clamp face and thus can increase friction. Under this condition, high-frequency current is applied on the wire (electrode) 18, and then the treatment part 22 is incised.

According to the high-frequency current forceps 25, the concentration of current density can be maintained by the concave portions 13c, reducing the likelihood of dispersion of the current density by a physiological saline solution, etc. In addition, the pair of clamp pieces 12 and 13 can clamp a tissue between them, reducing the likelihood of slipping. Therefore, an operation becomes easy because it is possible to clamp a living organ in a stable manner, and to firmly supply current on the desired treatment part 22.

While preferred embodiments of the present invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

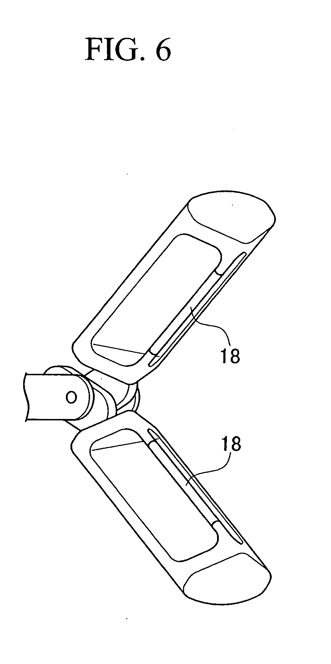

For example, although the clamp pieces 12 and 13 (a body 13a) according to the above embodiments are made of a metal such as stainless steel, etc., on which a surface is covered with the insulation film 17, an insulation material such as a ceramics, etc., may be adopted as the material instead of a metal. In addition, as for a high-frequency current forceps of which two ends of the clamp pieces have electrodes, the same action and the same effect can be obtained by adopting a constitution having the wire 18 supported on each of the ridgelines of the bodies as shown in FIG. 6.

Next, a forceps for an endoscope according to the related technology will be explained below. In the explanation below, for the same components explained in the above embodiments, the same reference numbers will be used and the explanation thereof will be omitted.

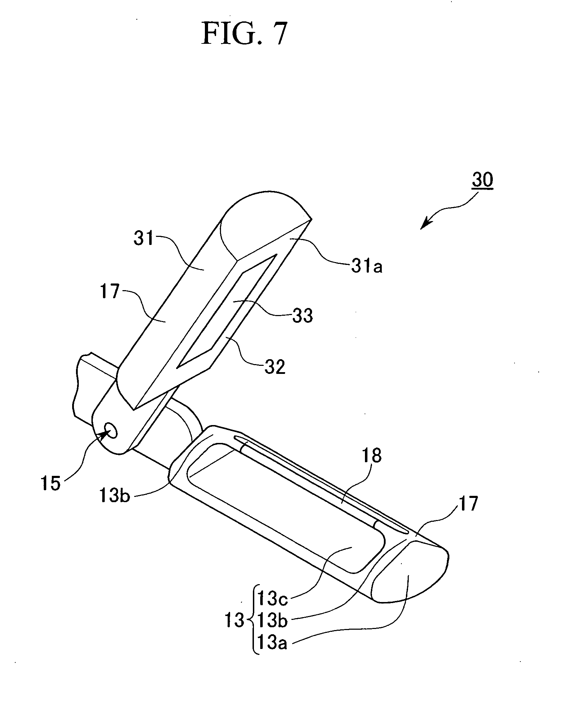

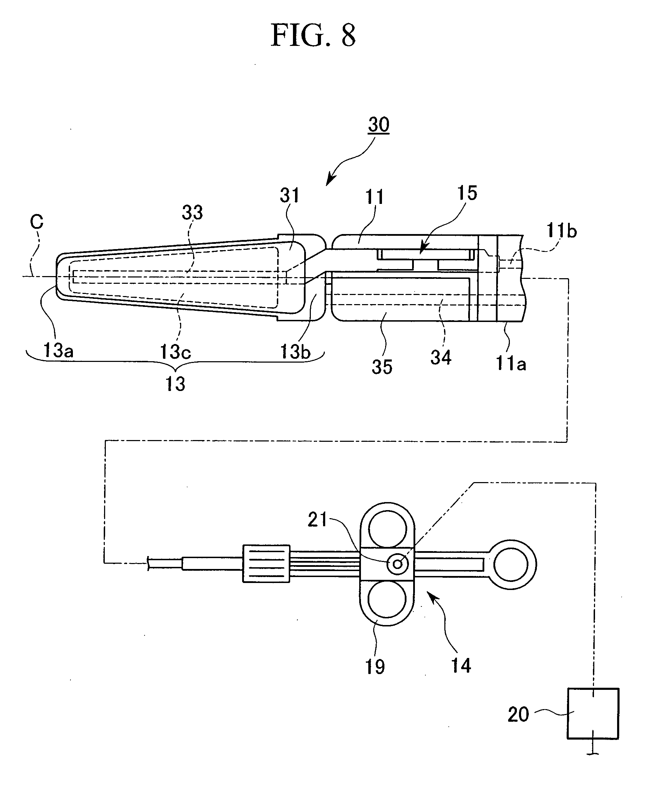

A high-frequency current forceps (a forceps for an endoscope) 30 according to the present related technology is, as shown in FIGS. 7 and 8, a bipolar type high-frequency current forceps which conducts incision, etc., of a living organ by supplying high-frequency current between the clamp pieces 13 and 31.

At the conventional bipolar type high-frequency current forceps, high-frequency current is supplied on clamp pieces provided on its tip portion by supplying the high-frequency current on a control wire which controls open-and-closing action of the clamp pieces.

A forceps is disclosed in FIG. 2 of the International Publication No. WO01/15614A1 which reduces the likelihood of current leakage by directly supplying high-frequency current on at least one of clamp pieces via an electric wire covered with an insulation material, but not via a link mechanism.

The high-frequency current forceps 30 according to the present related technology can easily have reduced diameter, and enables easy handling when inserting it into an endoscope and when removing it from the endoscope. Therefore, the high-frequency current forceps 30 can also be used together with a flexible endoscope.

At the high-frequency current forceps 30, as shown in FIG. 8, the clamp piece 13 is fixed on a tip portion of the shaft member 11. In addition, the clamp piece 31 is provided facing to the clamp piece 13, and only the clamp piece 31 is connected to the link mechanism, and conducts open-and-closing action.

The clamp piece 31 is made of metal such as stainless steel, and has a clamp face 31a facing to the clamp piece 13. As shown in FIG. 9, an electrode 32 formed by exposing a part made of stainless steel in the clamp piece 31, and a resin member 33 arranged on a location where the wire 18 contacts when closing between the clamp pieces 13 and 31, extending toward a length direction of the clamp face 31a, is provided on the clamp face 31 a. The resin member 33 is made of a fluoro plastic, etc., having an electric non-conductance, and the resin member 33 blocks direct contact between the wire 18 and the electrode 32. A surface of the clamp piece 31 except for the clamp face 31a is covered with an insulation film 17 which is electrically non-conducting.

The link mechanism 15 of the present related technology has the same constitution as in the first embodiment, but differs in that, as shown in FIG. 8, the link mechanism 15 of the present related technology is arranged to one side in relation to a center axis C of the shaft member 11, and the link mechanism 15 is joined to the control wire 11b.

One end of the wire 18 which is close to the ridgeline 13b supported on the clamp piece 13 is connected to the insulated wire 34 which is electrically insulated by being covered. The insulated wire 34 passes through a space 35 in the flexible tube 11a which is formed by placing the link mechanism 15 in one side in relation to the center axis C of the flexible tube 11a. Furthermore, the insulated wire 34 is placed inside the flexible tube 11a together with the control wire 11b, then is connected the high-frequency current power supply 20.

Next, use of the high-frequency current forceps 30 will be explained.

As same as the first embodiment, an endoscope having the high-frequency current forceps 30 is inserted into a body cavity. Next, the clamp pieces 12 and 31 clamp the treatment part 22 by operating the sliding controller 19, then high-frequency current is supplied to the control wire 11b and the insulated wire 34 by operating the high-frequency current power supply 20. At this time, the high-frequency current passing through the control wire 11b, the link mechanism 15, and the wire 18, reaches to the electrode 32 via the treatment part 22. Then the high-frequency current returns from the electrode 32 to the high-frequency current power supply 20 through the insulated wire 34.

Accordingly, the high-frequency current having very high current density is applied between the wire 18 and the electrode 32 through the treatment part 22 clamped between them, and the treatment part 22 is incised.

According to the high-frequency current forceps 30, as the link mechanism 15 is placed inside the flexible tube 11a together with the insulated wire 34 by placing the link mechanism 15 in one side in relation to the center axis C of the flexible tube 11a, the high-frequency current forceps 30 can adopt a narrow shape so that it can be used together with a flexible endoscope, and can improve operation ability when the high-frequency current forceps 30 is inserted into an endoscope and when the high-frequency current forceps 30 is removed from the endoscope.

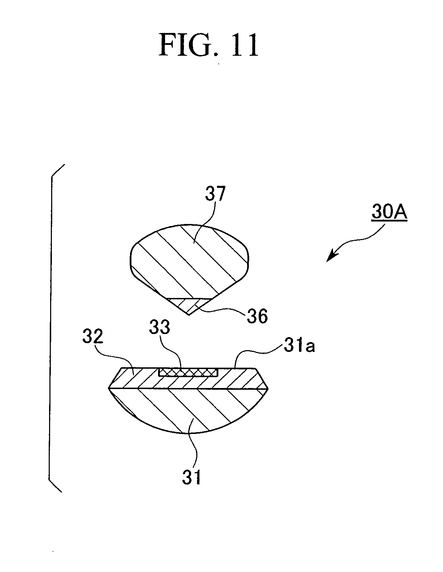

Moreover, as shown in FIGS. 10 and 11, it is possible to adopt a high-frequency current forceps (a forceps for an endoscope) 30A having the clamp piece 31 fixed to a tip end of the shaft member 11, and a clamp piece 37 facing to the clamp piece 31 and connected to the link mechanism 15. A linear electrode 36 is placed on a location of a clamp face of the clamp piece 37 facing to the clamp piece 31, where the linear electrode 36 faces to the resin member 33.

The high-frequency current forceps 30A can have the same effect because it can produce the same high-frequency current between the electrode 32 and the linear electrode 36.

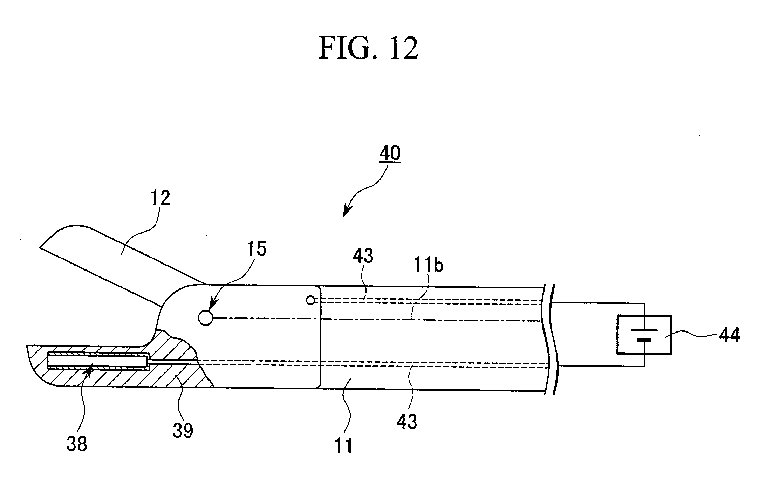

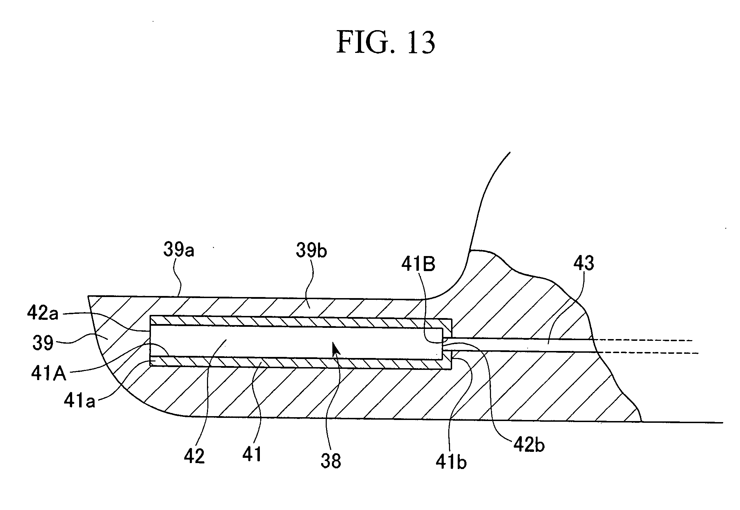

Furthermore, as shown in FIGS. 12 and 13, it is possible to adopt a forceps 40 having a clamp piece 39 in which a heat-emitting member 38 is placed, instead of the electrode 32 and the linear electrode 36. This forceps 40 can incise the treatment part 22 using heat emitted from the heat-emitting member 38 by supplying direct-current on it instead of supplying the high-frequency current.

In this case, the clamp piece 12 of the first embodiment can be applied as a clamp piece facing to the clamp piece 39. The clamp piece 12 is connected to the control wire 11b via the link mechanism 15.

An example of a forceps which incises a living organ using emitted heat is disclosed in, for example, FIG. 3 of Japanese Patent Application, First Publication No. 2001-340349.

The forceps 40 according to the present related-technology can have narrow shape and improved operation ability when inserting and removing it. Therefore, the forceps 40 can be used together with a flexible endoscope.

At the forceps 40, a ridgeline portion 39b is formed extending along a length direction of the clamp piece 39 on a center portion of a clamp face 39a of the clamp piece 39 facing to the clamp piece 12. And the heat-emitting member 38 is installed in the ridgeline portion 39b along the direction of the ridgeline portion 39b.

The heat-emitting member 38 includes a heater cover 41 and a heating element 42 installed inside the heater cover 41.

The heater cover 41 is made of an electrical insulation material such as ceramics, etc., and is formed extending along the ridgeline 39b. Furthermore, an opening portion 41 A for exposing one end 42a of the heat element 42 is formed on a tip end surface 41 a of the heater cover 41. In addition, a penetration hole 41 B is formed on a bottom end surface 41 b to connect between an insulated line 43 for electrical power supply and another end 42b of the heat element 42.

The insulated line 43 is placed inside the shaft member 11 together with the control wire 11b, and is connected to a direct-current power supply 44 together with the control wire 11b. Therefore, one end 42a of the heat element 42 is electrically connected to the clamp piece 39, and the another end 42b is electrically connected only to the insulated line 43 and is electrically insulated from the clamp piece 39.

Next, use of the forceps 40 will be explained.

As in the first embodiment, an endoscope (not shown in the figures) having the forceps 40 is inserted into a body cavity. Next, the clamp pieces 12 and 39 clamp the treatment part 22 by operating the sliding controller 19, then direct current is supplied from the direct current power supply 44 to the control wire 11b and the insulated wire 34. At this time, the direct current reaches the clamp piece 39 via the control wire 11b through a tip end side of the shaft member 11, then is supplied to one end 42a of the heat element 42 through the opening portion 41 A, then is returned to the direct-current power supply 44 from another end 42b via the insulated line 43.

Meanwhile, the heat element 42 emits heat by the direct current flowing inside it, then the treatment part 22 clamped between the clamp pieces 12 and 39 is heated and is incised.

According to the forceps 40, the shaft member 11 can be narrow so that it can be used together with a flexible endoscope because the heat element 42 is installed inside the clamp piece 39 together with the heater cover 41. Furthermore, operation ability for inserting and removing the forceps 40 in relation to an endoscope can be improved.

As explained above, according to the forceps for an endoscope of the present invention, current density can be increased because a surface area of the linear electrode contacting a living organ can be smaller in relation to a conventional forceps for an endoscope, by adopting the linear electrode. In addition, because the two ends of this linear electrode are supported on the body, only the treatment part facing the clamp face can contact the linear electrode. Furthermore, because the linear electrode is supported above the concave portion formed on the body, water leaking out from the living organ when clamping the living organ can be removed from the linear electrode through the concave portion. Therefore, decreasing of current density due to water can be prevented, thus concentration of the current density during an operation can be maintained.

By adopting a wire as the linear electrode, current density can be increased because the surface area of the linear electrode can easily be made smaller by adopting a smaller wire diameter. In addition, because the linear electrode and the body are separate parts, manufacturing of the clamp piece can be done easily.

Therefore, according to the forceps for an endoscope according to the present invention, because the linear electrode of which two ends are supported on the body of the clamp piece is provided, current density can be concentrated by adopting a linear electrode having a smaller surface area, and thus operation performance can be improved by easily and firmly operating only a treatment part which should be operated.

## Claims

1. A forceps (10) for an endoscope comprising a pair of clamp pieces (12, 13), wherein at least one of the clamp pieces comprises: an electrically insulated body (13a) on which a concave portion (13c) is formed on a clamp face of the body; and a linear electrode (18) having two ends supported above the concave portion.

2. The forceps for an endoscope according to Claim 1, wherein the concave portion is formed between a tip side portion and a bottom side portion of the body; and the two ends of the linear electrode are supported on the tip side portion and the bottom side portion.

3. The forceps for an endoscope according to Claim 2, wherein a ridgeline (13b) extending between the tip side portion and the bottom side portion except for the concave portion is formed on the clamp face, and the two ends of the linear electrode are supported on the ridgeline.

4. The forceps for an endoscope according to Claim 1, wherein the linear electrode is a wire.

5. A forceps (25) for an endoscope comprising a pair of clamp pieces (12, 13), wherein at least one of the clamp pieces comprises: an electrically insulated body (13a) on which a plurality of concave portions (13c) are formed on a clamp face of the body along a crossing direction in relation to a direction extending between the a tip side portion and a bottom side portion of the clamp face; and a linear electrode (18) supported above the concave portions.

6. The forceps for an endoscope according to Claim 5, wherein the linear electrode is a wire.
